# EUROPEAN PATENT SPECIFICATION

(11) **EP 3 448 300 B1**
(45) Date of publication and mention of the grant of the patent: **27.05.2026**
(21) Application number: 17789995.2
(22) Date of filing: 28.04.2017
(51) Int. Cl.: A61C 8/00, A61C 13/08, G06T 17/00, A61C 13/00

(54) **BIONIC IMPLANTS**
BIONISCHE IMPLANTATE
IMPLANTS BIONIQUES

(30) Priority: 28.04.2016 PL 41702816
(43) Date of publication of application: 06.03.2019
(73) Proprietor: Af Praktyka Stomatologiczna Artur Frydrychewicz, 02-123 Warszawa (PL)
(72) Inventor: FRYDRYCHEWICZ, Artur, 02-123 Warszawa (PL)
(86) International application number: PCT/PL2017/000045
(87) International publication number: WO 2017/188832

(56) References cited:
- WO-A1-2005/079696
- WO-A1-2014/181144
- CN-A- 104 352 285
- CN-A- 105 327 400
- CN-U- 205 094 649
- CN-Y- 201 404 302
- DE-A1- 3 723 643
- JP-A- H08 196 547
- US-A- 2 721 387
- US-A- 5 006 070
- US-A- 5 873 721
- US-A1- 2007 264 612
- US-A1- 2009 258 329
- US-A1- 2010 055 646
- US-A1- 2013 209 961

## Description

The present invention relates to bionic implants, especially for dental applications, according to the appended claims.

Implants consist of a head, a core (corpus) and an anchor. The core is equipped with a prosthetic platform, which allows for the attachment of prosthetic or secondary components.

Traditional endosseous implants include the following: cylindrical and conical screw-type implants; threadless cylindrical implants; disc implants, needle implants and blade implants. The names assigned to these different groups of implants are derived from the shape of the anchor, i.e., the part of the implant that attaches to the bone. Their common feature is a simple geometric form usually designed to ensure ease of manufacture using traditional material processing methods (milling, threading, sheet metal cutting). Implants can be single-component or multiple-component devices. The material traditionally used to manufacture implants is titanium, while the newest implants are made from zirconia or synthetic materials, e.g. PEKK - polyetherketoneketone. Most of these implants are inserted in the alveolar process by way of screwing the implant anchor into the bone.

The most popular implant anchors currently take the form of a cylindrical or conical screw. The latter tapers towards the bone facilitating the screwing in of the implant, e.g. KR200416306(Y1). To impede implant rotation around the longitudinal axis, various notches, indentations, and intersecting holes are used, as is for example shown in patent specifications CN101297772 (A), CN1565390(A).

The newest types of implants are bionic and biomimetic. For the needs of this description, bionic implants are defined as implants designed with inspiration from functions of objects present in nature. By way of contrast, biomimetic implants mimic the structures they replace. The best-known biomimetic implants imitate the shape of a tooth's roots (e.g. Replicate or Bioimplant). Most frequently, an anchor of this type differs from a pure replica of a tooth root in that its external surface possesses indentations or protrusions - these serve to increase the anchor's surface area, facilitate its embedding in the bone and create space for bone growth. Implants like these are only used in immediate implant placement procedures, and they are designed exclusively for making cemented restorations. Prior to the procedure, a structure approximate to that of the tooth root it is intended to replace is created based on analysis of tomographic imaging. Such anchors are manufactured by means of milling or CAD/CAM (Computer Aided Design/Computer Aided Manufacture) or SLM (Selective Laser Melting) methods.

The idea of bionic implants is to create a broad base for the implant so as to ensure greater stability and allow for many buttress points. With this aim in mind, attempts have been made to use needle implants whose anchors consist of separate needles, which, after being independently inserted into the bone, are then attached to the core by means of gluing or welding.

There are solutions where the implant resembles a tooth root but also possesses a regular geometrical shape, such as the implant shown in utility model CN203468769 (U). The anchor of this implant is shaped like a cylinder, which branches out into two arms with a uniform cross-section along their entire length.

A slightly different example of a bionic implant is that of the multi-root implant described in patent specification US2009061387 (A1). Emerging from the core are 2 - 4 separate, regularly shaped roots that taper towards the apex but have blunt ends. The number of the implant anchor's roots corresponds to the number of roots of the tooth that the implant is replacing. Subperiosteal implants (placed on top of the bone) that recreate the bone surface can also be called bionic implants. They were once made from a bone model prepared after taking impressions (a highly burdensome procedure) and using the lost-wax technique. They are currently made by use of tomographic imaging (3D) models, using CAD/CAM or SML methods.

A partially subperiosteal implant is shown in patent specification KR101469648 (B1). Here the implant has a titanium coating in the form of a mesh with openings for screws. The titanium mesh is placed on osseous tissue, thereby reproducing its shape, and soft tissue is placed on top of the titanium mesh. The screws are screwed into the bone in order to ensure attachment of the titanium mesh, which also includes rings of a prosthetic platform.

If it is necessary to recreate the root system of a multi-rooted tooth whose roots branch outwards creating a wider base deep in the bone tissue, solutions involve the construction of anchors consisting of at least two parts that are attached during implant placement in order to avoid excessive drilling in the post-extraction socket.

In accordance with CN 100571651 C (WO2008125049 (A1)), holes are drilled in an anchor whose main part is screw-shaped, and during implant placement arms are placed in these holes at an acute angle to the axis of the main part of the anchor. The arms possess a regular cylindrical shape finished with a screw section. An implant anchor composed of two regularly shaped parts whose position corresponds to the branches of the tooth's roots is also shown in utility model CN201404302 (Y). A similar idea inspired the construction of the bionic implants described in patent applications US20130288201 (A1), US5984681 (A) and CN101249023 (A). A major drawback of the solution in which the anchor consists of two or more parts and is attached as a whole in situ, is that there is a risk of the formation of micro-gaps, through which bacteria can make their way to the bone, thereby causing inflammation around the implants (peri-implantitis).

Also known in the prior art is US2721387A which relates to artificial teeth, particularly to one which is adapted to be inserted in the socket of a tooth which has just been extracted so as to take the place of the extracted tooth, and to be retained in the position of the extracted tooth without bridging or other support from the proximal or other teeth in the denture.

Numerous modern radiographic techniques have contributed to advances in implantology, especially computed tomography, CT (currently the most recommended approach is cone beam computed tomography - CBCT, due to its reduced radiation dose)._In radiology, analysis of tomographic imaging utilises layers. It is possible to obtain an image of a layer of any section of the body, e.g. the head. In dentistry, CT scans are taken of the maxilla and the mandible. At present, the minimal layer thickness (slice) that can be achieved with advanced tomographic imaging device is 70 µm - the same as the voxel size used in such devices. The maximum thickness of a layer is the full scanning range (the summation layer). Traditional radiology distinguishes between three basic planes: frontal, sagittal and axial. These correspond to the position of the main tomographic layers - orthogonal (straight) layers - while the software currently used for analytical purposes also allows for layers that diverge from traditional planes by the angle desired by the user, producing oblique layers. It is also possible to produce a slice image comprised of consecutive short fragments of oblique layers at a tangent to the so-called panoramic curve. Panoramic curves are drawn arbitrarily with regard to objects of interest. This makes it possible to obtain a planar image of any given cross-section, known as the panoramic layer. The panoramic layer is used to create an image derived from computed tomography (CT), which is similar to a traditional pantomogram, also known as a panoramic image. Basing on the panoramic layer, cross-sections perpendicular to the panoramic layer are also obtained. This makes it possible to assess the availability of tissue essential for proper implant placement prior to performing the procedure, and ensures better preparation for planned procedures by allowing for the virtual superimposition of currently used implant models onto the image, i.a. This aids the selection of the right size of the implant.

Another modern implantological method is guided implant placement, which is currently employed by many manufacturers. This approach involves using tomographic imaging and 3D printing to create surgical templates. Its purpose is to achieve precise implant placement, which requires ensuring the implants position in the bone is set precisely with regard to both the surrounding tissue as well as future prosthetic restorations, using tomography analysis software. In order for implant placement to proceed according to plan, a 3D printed template should be prepared detailing how surgical instruments (for example, drills) and the implant/implants should be inserted.

The use of traditional implants is associated with many problems.

The biggest problem in implantology is that of bone deficit, for if a suitable margin of bone tissue is not preserved, primary stability cannot be achieved. The matter is further complicated by sensitive anatomical structures, such as nerves, passing through the bone. In the case of the mandible, the current state of the art offers two ways of solving this problem. The first method is bone augmentation, which involves "building" bone above the nerve canal. This is a complicated procedure that carries a risk of complications and a significant increase in costs due to the need for additional tools and materials. The second method is nerve lateralisation, which entails surgically cutting through the bone and repositioning the nerve. Once again, however, this is a risky procedure, which can lead to nerve damage, on top of which the scope of the procedure itself is very extensive. Both methods prolong the course of treatment due to the additional rehabilitation time required. In the case of the maxilla, implant placement requires augmentation procedures (a sinus lift, a bone graft from the iliac crest or other techniques of alveolar ridge reconstruction), which in turn have undesirable consequences. This often leads to situations where the patient is reluctant to undergo treatment. One compromise approach is to use threaded implants of small dimensions, which results in large unit loads on the contact surface between the implant and the bone. Bone remodelling as a result of increased loads leads to osseodensification and a decline in volume, which causes implants designed as endosseous implants to function as partially subperiosteal implants that remain only partially embedded in the bone. This is particularly disadvantageous in cases where the geometry of the implant creates sharp edges (for example, thread), which because they are not immersed in the bone cause harm to soft tissue, thereby damaging it and often causing inflammation, i.e. peri-implantitis.

This invention introduces a new approach to the construction and functioning of bionic implants.

The implant design according to the invention has been presented in claim 1. Dependent claims 2 - 7 describe various additional optional features whereby each feature being the essence of the subsequent dependent claim characterizes an example of embodiment.

The manufacturing of a standard implant, the structure of which is defined by claims 1-7, entails the use of 3D design software to create a virtual implant that is printed in a 3D printer and then subjected to finalising processes. In the current state of the art, only customised implants are made with a 3D printer. Bionic arms are, within the understanding of the present invention, arms modelled on biological forms, inspired by shapes and forms present in nature, such as claws, teeth (e.g. shark teeth), pointed ends (claws of predators), fingers, thorns, catching elements (e.g. burrs), roots, mycelial chords, scales, and bulbs. The observation that nature adjusts form to function regardless of the scale is utilised here. Thus, when designing bionic implants in accordance with this invention, the aim was also to apply the rotational odd symmetry existing in the plant world in order to increase durability. Attention was also paid to the possibility of using biological development processes (morphogenesis) with the aim of seeking new forms. Digital morphogenesis enables transformations that adjust form and structure to the needs of utility. This, in turn, makes it possible to build complex, curvilinear spatial structures by increasing construction parameters.

The bionic anchor can serve as both an endosseous and a subperiosteal implant, due to the presence of these arms. Simultaneously, primary stability increases due to loading.

In accordance with the present invention, the implant anchor's arms possess a number of functions and associated advantages. These include increased anchor surface area; primary implant stability enhanced by the elimination of rotation; the establishment of an implant insertion trajectory by cutting and wedging into the bone; bracing against the bone; wedging that prevents deeper immersion, and buttressing the bone. The arms can either perform certain functions, or a single arm can perform several different functions, such as acting as a sharp end for intruding into the bone, or inhibiting further intrusion with a bulge on the proximal part of the implant.

For the needs of the present invention, panoramic surfaces are defined using the concepts of the panoramic curve and panoramic layer used in radiology. The simplest panoramic surfaces (identical to panoramic layers with hypothetical zero thickness) are obtained when parallel lines pass through panoramic curves, which slice the spatial image, and the surface of the intersection spreads out over a flat surface to produce a flat image of the intersected interior. A panoramic curve may consist of straight sections, with the panoramic surface then constituting a kind of multi-plane cross-section.

In contrast to panoramic layers, panoramic surfaces can be formed in a different and more complicated way. A panoramic surface can also be created on the basis of triangles joining two or more panoramic curves.

The basic criteria for determining panoramic curves and surfaces are: the exposition of bone volume available for placing the implant anchor's arms; the possible avoidance of sensitive structures and optimisation of the anchor's surface to ensure implant stability.

In accordance with the present invention, the anchor allows for more gradual anchoring in the bone as well as a gradual transition over time from functioning as an endosseous implant to functioning as a partially subperiosteal implant. The arms are also used to model space for tissues around the implant. They also make it possible to adjust the geometry of the implant to the shape of any bone defect.

As was mentioned above, the arms of the implant anchor can provide space for bone reconstruction. The cutting edges and spikes of the arms act as retention elements that ensure primary stability. They serve as an anti-rotational, supporting function and take on the main preliminary load during the healing stage.

This invention is especially suited for one-stage implants as well as for implants that feature an internal prosthetic platform in their core.

The implants in accordance with the invention perform well in cases of immediate implant placement as well as in delayed procedures, in extremely shallow and deep implant placement procedures, in both customised and standardised procedures. They open up new possibilities for developing surgical techniques. It is possible to piezosurgically prepare the implant bed using cutting edges identical to the implant, to place implants by means of hammering (manual or aided with certain instruments, e.g. a magnetic mallet). There is a trend regarding the placement of increasingly shorter implants. The present invention enables implant placement in difficult anatomical conditions without the need for bone augmentation. By virtue of this invention, the axis of attachment of prosthetic restorations or secondary elements is independent of the axis of implant placement.

Implants in accordance with the invention can be manufactured using traditional methods or with the latest CAD-CAM or CAD-SLM software. All that is needed to manufacture the invention are zirconia, titanium, PEKK, and Ti alloys - materials that are widely used in implantology. Likewise, the surface can be finalised using well-known techniques.

The invention allows for lower costs and advances the availability of personalised solutions in medicine.

There are also other benefits involved with the use of this invention: there is no need for an expanded internal interface (connection); there are fewer faults; the elimination of areas of bacterial retention; and the possibility to achieve multidimensional stability (multi-point buttressing). This invention facilitates primary and secondary stability, and it reduces tension in the bone surrounding the implant, owing to a favourable distribution of forces.

Simultaneously, this invention does not determine the formal embodiments of the crown and core.

The object of the invention as presented in its embodiments will be explained using a schematic diagram, whereby:
Fig. 1 shows the first embodiment of a standard implant;
Fig. 2 shows the second embodiment of a standard implant;
Fig. 3 shows the fourth embodiment of a standard implant;
Fig. 4 shows the fifth embodiment of both a standard implant and an individual implant;
Fig. 5 presents different versions of a variation of a standard or individual implant;
Fig. 6 shows an example of an implant with cross-sections of the anchor at different heights;
Fig. 7 (a,b,c) shows a view of sample arms of an implant anchor;
Fig. 8 shows an implant that uses the arms from Fig. 7;
Fig. 9 shows sample views of cross-sections of implant anchors;
Fig. 10 shows examples of different individual arms of implant anchors;
Fig 11 shows cross sections of sample arms;
Fig. 12 (a,b,c), show implants with different forms of anchors;
Fig. 13 (a,b,c,d) show implants with different forms of anchors;
Fig. 14 shows a cross-section of the alveolar process by the maxillary sinus with the implant as well as views of different embodiments of an implant anchor suitable for such a case;
Figs 15, 16 show a view of the maxilla and mandible, respectively, with examples of implants drawn in as well as the anchors of these implants.
Figs 17-18 are presented only for explanation of the new method which is not a part of the present invention.
Fig. 17a shows a perspective view of a fragment of the mandible with a cut out panoramic surface, Fig. 17b presents the view from above of a fragment of the mandible from Fig. 17a, Fig. 17c shows the panoramic surface from 17a. on a plane;
Fig. 18a shows a perspective view of a fragment of the mandible with a cut-out of the panoramic surface, Fig. 18b shows the view from above of a fragment of the mandible with Fig. 18a, Fig. 18c shows the panoramic surface with Fig 18a on a plane;
Fig. 19a shows a view from above of a fragment of the mandible with a drawn implant anchor, while Fig. 19b shows a fragment of the mandible with implant anchor in perspective view.
Fig. 20 shows the view from above of a fragment of the mandible with implant anchor in another embodiment.

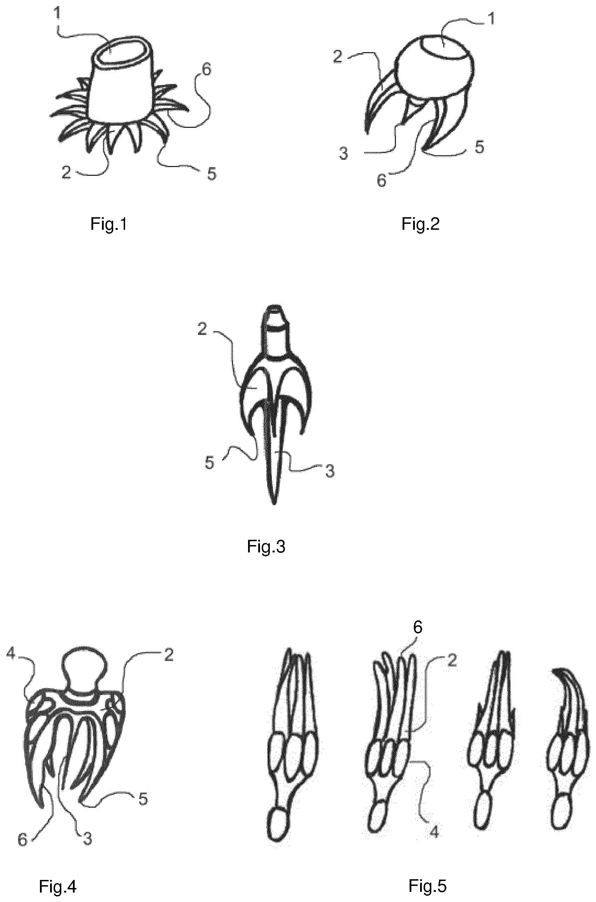

Figs 1-3 show examples of standard implants, which can be calibrated to create a range of implants ready for placement. In Fig. 1 the anchor is constructed out of a row of arms 2 in the shape of short claws finished with pointed ends 5 as well as cutting edge 6. This kind of implant can be anchored at a very shallow depth, e.g., at 2 mm. Even this slight embedding of many short arms in the bone suffices, on account of the largest possible anchor surface achieved in such conditions and the relatively large area of the implant base, thus ensuring a high level of primary stability. In Fig. 1 as well as Fig. 2 the core of the implant possesses a dome-like concavity 1. This makes it possible to place different materials, such as elastomer and ferromagnetic materials, inside the implant. Fig. 2 shows an implant with a standard structure and a greatly simplified geometry. It has a spherical core possessing a dome 1 and a central spike 3 as well as three sharp arms 2, which will pierce the bone like a thumbtack. In the case of this embodiment, implant placement ensures maximum simplicity. It is also suitable for temporary implant placement procedures, as the removal of such an implant would be simple, especially if fewer arms were used and the arms' surface is smooth.

The implants presented in Figs 1 and 2 are suitable for cases where the bone cannot be prepared too deeply, e.g. only at 2 mm. In the current state of the art, the problem has been to achieve stability, especially primary stability. Screw-type implants are not very well suited to cases where the insertion depth is less than 4 mm. In the current state of the art, screws with a large thread (disc) diameter are used, although they have a significantly smaller surface than that achieved by the anchor in accordance with the invention. In the same conditions, if we used the implant with the arms specified in the present invention we could achieve a much greater surface for the implant in the bone, which results in high primary and secondary stability parameters.

The anchor of the implant from Fig. 3 possesses side arms 2 in the form of claws and a much longer spike 3, which makes it possible to set the original axis of placement. The arms 2 are tapered to form an arch. It is not necessary for all the side arms 2 to be stuck into the bone. A large amount of space is left around the implant anchor with this aim in mind so that the bone can grow into the space between the arms of the anchor, and at the edges of the side arms 2 we achieve support against the bone. During the healing stage the bone will also be able to grow above the anchor.

Fig. 4 shows the anchor of the implant intended for the alveolus following extraction of a lower molar. An implant of this kind is designed for immediate implant placement procedures. Located between the roots of the molar is the interradicular septum. The implant anchor has a central spike 3, which after extraction of the tooth punctures the interradicular septum. The central spike 3 does not damage the septum in the same way as a screw-type implant (in the current state of the art), which requires drilling a hole with a much greater diameter than the diameter of the hole formed after inserting the central spike 3. Penetration of the interradicular septum ensures primary stability and determines the trajectory of the implant placement axis. The other arms 2 that enter the alveolus are significantly more branched than the original natural tooth or any kind of traditional implant used in such cases. These branched arms 2 increase the surface of bone apposition and the internal volume of the body of the implant into which the bone grows. The arms 2 also possess cutting edges 6 directed towards the interior of the implant body. These cutting edges 6 also add primary stability by cutting from the outside into the interradicular septum stretched by the central spike 3. The sharpened tips of the arms 2 - pointed ends 5 - are anchored at the base of the alveolus. In the lower core section the arms possess protrusions 4. These ensure that not too much pressure is placed on the bone in the zone where the bone is thinnest and the target loads are greatest. The complexity of the anchor surface in accordance with the specifications of the invention enables greater integration of the implant anchor with the bone than is the case with a traditional implant based on the current state of the art.

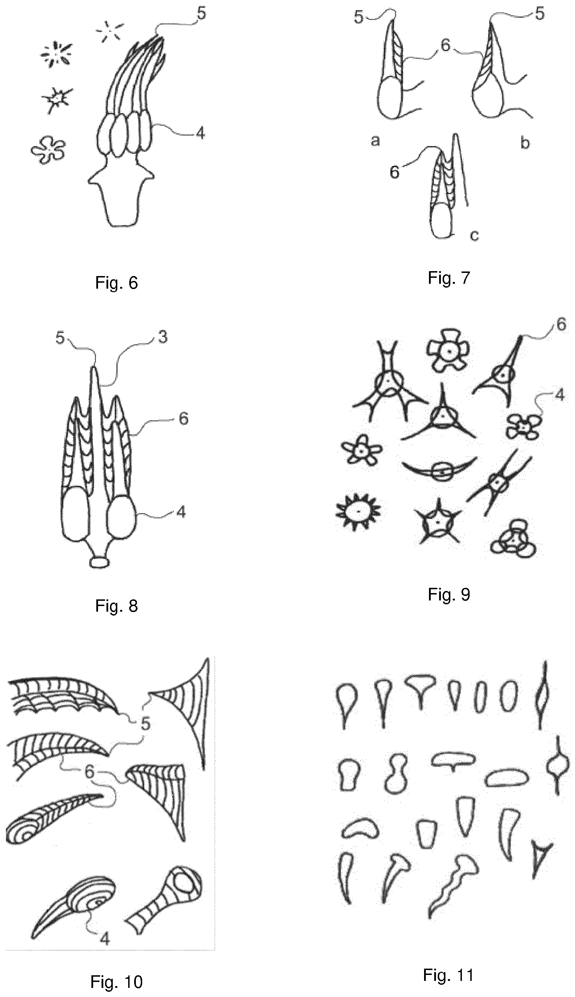

Figs 5, 6 and 8 show other examples of implant anchors for use in immediate implant placement. The implant anchor is to a certain extent modelled on the patient's own tooth, but differs from the original teeth in that it possesses arms 2, which form pointed ends 5 or cutting edges 6 at the root apex, and protrusions 4 in the cervical section. Each of the side arms 2 can branch out into, e.g. two more arms whose cutting edges 6 can adjust the trajectory of implant placement. Moreover, if the anchor also has side arms 2 with cutting edges 6 at the edges, as in Figs 7 and 8, it can cut into the walls of the alveolus with these cutting edges 6, thereby creating additional stability. On the other hand, in the region where healing conditions should be as stable as possible there are no cutting edges only smooth surfaces - protrusions 4, thereby resulting in reduced alveolar filling. During the course of remodelling, the bone tissue grows into the space between the arms 2 in the area of the largest secondary loads. In addition, in this area, the arms 2 remain immediately after implantation in gentle contact over a larger surface with bone tissue at the tops of the protrusions 4, spreading the alveolus below the bone itself, allowing tissue healing without compression while preserving their original outline. The elasticity of the bone ensures an even distribution of pressure along the circumference of the alveolus.

Fig. 7 shows examples of arms, which can be used in anchors of the type presented in Fig. 5, Fig. 6 and Fig. 8. Visible protrusions 4 pass through the thickened section of the arm, thereby ensuring rigidity of the structure, while further on is part of the arm, which features cutting edge(s) 6. Protrusions 4 are responsible for taking the greatest secondary loads - here secondary stability will result from bone apposition. Cutting edge 6 in the examples presented in Fig. 8 7 is located either inside the body of the implant (towards the interradicular septum of the alveolus - example 7a), or directed outside the body of the implant towards the alveolus - example 7b) or one arm features two cutting edges pointed in opposite directions, connected with a cutting edge running through the apex of the arm (example 7c). For example, 7c shows a situation where the greater part of the cutting edge pointed towards the interior of the implant body connects with the core of the implant.

Fig. 8 shows an implant capable of replacing, for example, a two-rooted tooth or a single-rooted tooth with an elliptical cross-section of the root. The implant anchor possesses the arms from Fig. 7c. In this example, it is equipped with a central spike 3 situated on the axis of the implant core. Its role is to stabilise the implant path of insertion, giving the latter path a direction. In the example from Fig. 8 the arms interpenetrate, forming in this way a common area - we can see the exterior contour.

Fig. 9 presents views of cross-sections of implant anchors; these are cross-sections in relation to the axis of the implant core. The anchors of the implants are multi-armed. The cross-sections are at different heights and the side cutting edge 6 are visible, as are protrusions 4.

Fig. 10 presents examples of different individual arms with a variety of complex shapes. The arm profile can be convex, concave or convex-concave.

The arms of the implant anchors in accordance with the present invention vary greatly, just as the needs are different on account of differences in the structure of maxillary and mandibular bones. The arms of implants ensure optimal use of bone volume by maintaining low unit pressure as well as integration with the bone tissue, thereby allowing for its stimulation with even loads. When the implant is inserted some cutting edge 6 perform the role of fins the moment they come into contact with the bone - the movement of the whole implant will additionally be controlled and directed in accordance with the direction of these cutting edges. These cutting edges 6 can thus be used to control and correct the performance of the implant while it is being placed in the bone. The finite element method applied to these arms and to the anchor or implant as a whole ensure optimal configuration of the geometry of the arms and anchor from the viewpoint of structural analysis and bone tissue penetration dynamics.

Fig. 11 shows examples of cross-sectional shapes of particular arms, where some of these cross-sections may be cross-sections of the same arm at different heights. In other words, at one end an arm is sharp on one side and rounded on the other, and above it the cutting edge becomes increasingly blunt until the end is rounded on both sides. The arms can assume many different shapes.

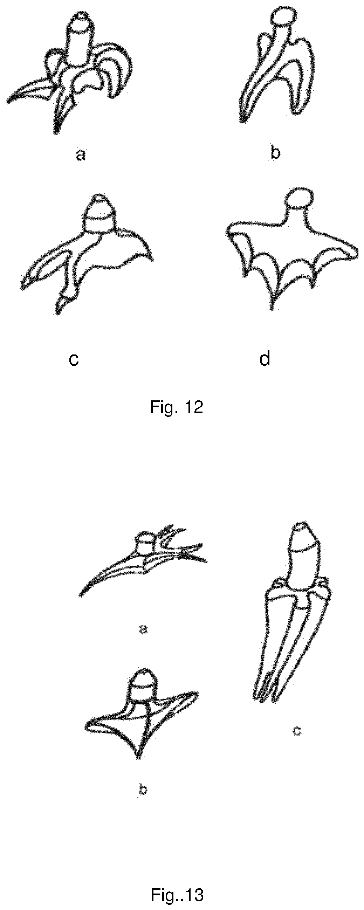

Figs 12 a, b, c and 13 a, b, c, d show many different forms of implant anchors in accordance with the present invention. The different shapes of anchors are suitable for different cases of implant treatment, depending on the shape of the alveolar processes and accessible depth of implant penetration. Fig. 13a shows the anchor of an implant suitable for shallow embedding, Fig. 13b shows an implant anchor suitable for cases involving a narrow alveolar process. Both examples are used with the osteodistraction technique (arms placed in the distraction gap). Fig. 13c shows an implant designed for immediate application as a replacement for a single-rooted tooth. Fig. 13d shows an implant intended for immediate application as a replacement for an upper molar.

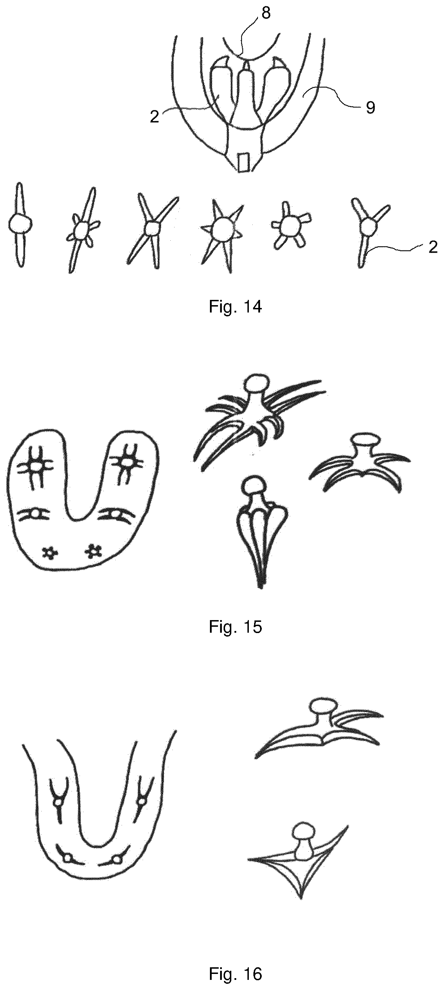

Fig. 14 shows a cross-section of the alveolar process with a placed implant, where the base of the maxillary sinus 8 and soft tissue 9 are also marked. This example is applicable when there is little space in the maxilla deep in the alveolar process on account of the danger of penetration into the maxillary sinus 8. Also shown are examples of cross-sections of anchors with arms 2 intended for application in such cases. They make it possible to utilise the volume of bone in each direction through various configurations of the arms, which can be inserted into the bone by increasing the implant anchor surface. One distinctive feature is that the implant core itself remains at a distance from the sinus base, which is not possible with traditional screw-type implants and requires either additional procedures or runs a risk of the implant penetrating the sinus. Even if such screw-type implants do not break through during placement, but are set at a shallow depth under the base of the sinus, they cannot be subjected to significant loading before osseointegration.

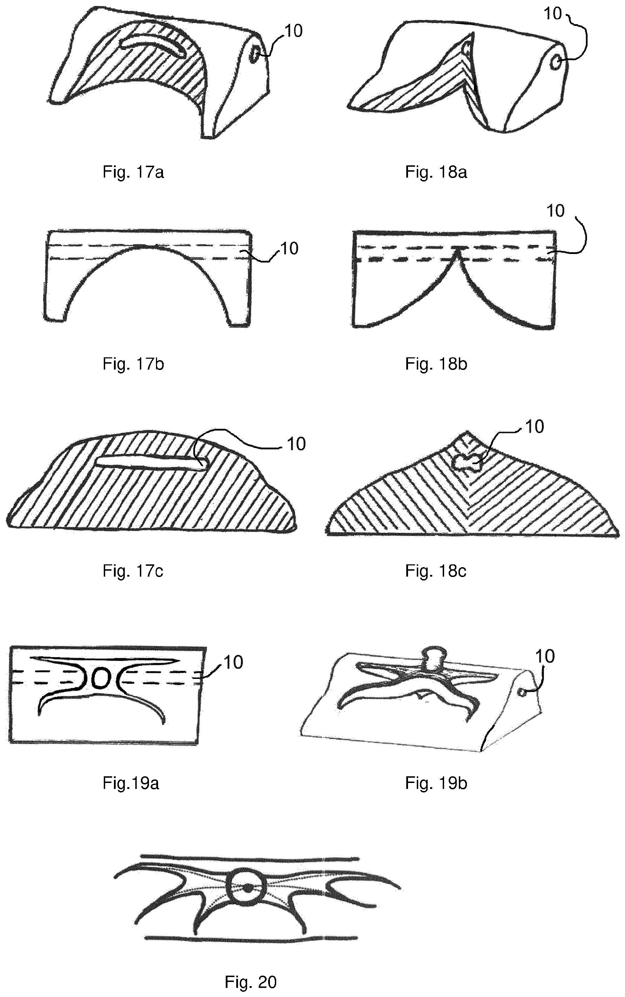

Fig. 16 shows an example of the distribution of different forms of implants in an edentulous maxilla as well as examples of possible forms that such implants can take. Similarly, Fig. 17 presents an example of the distribution of different forms of implants in an edentulous mandible as well as a view of such implants. The use of these forms together with their location allows us to optimise the distribution of forces under prosthetic restorations functioning as full dental arches.

Figs 18a and 19a show diagrams offering a perspective view of the tomographic image of a fragment of the mandible illustrating the course of the mandibular canal 10 (the course of the nerve and blood vessels), where certain fragments of these images have been cut out with different panoramic surfaces applied.

In the current state of the art, as was mentioned prior, layered cross-sections, which can be treated as panoramic surfaces if they have low thickness, are used for analysis of environmental conditions for implant placement. These cross-sections usually run along the length of the bone (and thus also the course of the mandibular canal), indicating insufficient space for traditional implants in cases of bone deficit.

Figures 18-19 do not belong to the present invention but just explain a new approach to bone analizing method in design of bionic arms shape for custom implants optimalization.

Figs 17a and 18a show the construction of panoramic surfaces in two ways. In Fig. 17a the panoramic surface is a surface based on a semi-circular panoramic curve. Fig 17b shows the view of such an intersection from above, while Fig. 17c illustrates the resulting panoramic surface achieved after its positioning on a plane. Such a panoramic surface creates an image similar to a traditional panoramic layer and would suggest that the nerve canal is a barrier almost impossible to avoid without performing additional procedures. In the search for a convenient panoramic projection of the arms of the anchor, this schematic fragment of the mandible was virtually intersected by a surface spread out on parallel lines drawn on a curve formed from two quarters of circles positioned as mirror images of each other, as is visible in Fig. 18a. In both cases, for simplicity's sake the panoramic curves are elements of simple geometric shapes. Just as in Fig. 17b, Fig. 18b presents a view from above of a simplified fragment of the mandible with a panoramic curve and similarly to Fig. 17c, Fig. 18c shows the panoramic surface laid out on a plane obtained as a result of the above-described cut. In the example illustrated in Figs 18a, 18b and 18c the nerve canal is visible in a very small section at the site of the planned core. Only along a short section of the implant anchor does it extend above the bone, as a result forming a subperiosteal "bridge," on which the core will be placed. A further part of the cross-section shows space free from hindrances within the bone tissue where the anchor arms can take shape freely.

And thus, in accordance with the new method which is not a part of the present invention, in cases where, for example, the nerve canal is positioned in such a way that no room is left for the use of traditional implants, the panoramic surfaces can be arranged so as to construct an implant whose arms bypass the nerve and run parallel to it, even in quite close proximity, although essentially at a safe distance, thus ensuring a high level of implant stability.

In accordance with the method, panoramic surfaces are guided arbitrarily aiming to find the most convenient possible course for the anchor arms. Through the use of this procedure, we also have the possibility of dimensioning the available space to create an implant anchor with individual dimensions.

An anchor designed with the panoramic surface from Figs 18a, b and c is presented in Figs 19a and 19b. Fig. 19a shows the view from above of a fragment of a mandible with an implant anchor in place, while Fig. 19b presents a perspective view of a fragment of the mandible with the implant anchor in place. It can be seen that the core is located directly above the nerve canal, without, however, endangering it. The arms 2 of the anchor will run on both sides of the nerve canal 10, penetrating deeper in those places where the volume of bone is greater - just as the roots of a plant grow in soil while bypassing stones.

Fig. 20 presents in diagram form a projection of another sample implant on a plane perpendicular to the axis of the core against the background of the bone. Such an implant is suitable for cases of edentulism, even when there is extreme alveolar atrophy. The implant anchor possesses two arms that in turn branch out into three further arms, where the point on the axis of the core is the place where the panoramic curves intersect, as marked with dotted lines. The arms of the anchor are described on panoramic surfaces that begin on these panoramic curves.

Using the above described methodology as well as tools in the form of modern diagnostic imaging methods (e.g. computed tomography (CT) or magnetic resonance) together with 3D printing we are able to create functionally optimised implants owing to their bionic anchor arms.

## Claims

1. A customized or standard bionic implant, especially for dental applications, containing at least one single-component anchor and at least one rounded core, and which is equipped with a prosthetic platform, wherein the anchor possesses at least two arms (2) whose axes are curved and extend from a point on a core axis **characterised in that** the at least two arms (2) are bionic arms (2) for bone penetration that taper circumferentially, that is away from the core, thereby creating at least one blade (6) at each arm, wherein the arms have blades at their edges, and the blades can cut into the walls of the alveolus, thereby creating additional stability.

2. The implant according to claim 1, **characterised in that** the at least two bionic arms (2) further comprise sharpened tips.

3. The implant according to claim 1, **characterised in that** at least two bionic arms (2) interfuse to form an integrated volume.

4. The implant according to claim 1, **characterised by** one additional arm finished with a pointed blade (5) that constitutes a central spike (3)

5. The implant according to claim 1, **characterised in that** the axis of the implant core is a curved line.

6. The implant according to claim 1, **characterised in that** a protrusion (4) is present on a fragment of each bionic arm (2).

7. The implant according to claim 1, **characterised in that** the rounded core possesses a concavity (1), which serves as a dome that can be filled with curable material, wherein the cross-sectional area of the dome opening is smaller than the cross-sectional area of its midsection.

## Patentansprüche

1. Ein individuell angepasstes oder ein bionisches Standardimplantat, besonders für stomatologische Anwendungen, welches mindestens einen einteiligen Anker und mindestens einen abgerundeten Implantatkörper enthält und welches mit einer prothetischen Plattform ausgestattet ist, wobei der Anker mindestens zwei Arme besitzt (2), deren Achsen krumm gebogen sind und sich vom Punkt auf der Achse des Implantatkörpers erstrecken, **dadurch gekennzeichnet, dass** mindestens zwei Arme (2) bionische Arme (2) zum Eindringen in den Knochen sind, die sich umlaufend, also in Richtung vom Implantatkörper weg, einengen, indem sie auf diese Art und Weise mindestens eine Schneide (6) auf jedem Arm bilden, wobei die Arme diese Schneiden auf ihren Kanten haben und die Schneiden sich in die Wände des Zahnfaches einschneiden können, indem sie auf diese Art und Weise eine zusätzliche Stabilität gewährleisten.

2. Das Implantat nach Anspruch 1, **dadurch gekennzeichnet, dass** mindestens zwei bionische Arme (2) darüber hinaus spitzige Endteile aufweisen.

3. Das Implantat nach Anspruch 1, **dadurch gekennzeichnet, dass** sich mindestens zwei bionische Arme (2) verbinden (einander durchdringen), indem sie einen integrierten Körper bilden.

4. Das Implantat nach Anspruch 1, **gekennzeichnet durch** einen zusätzlichen Arm, der mit einer Punktschneide (5) abgeschlossen ist und der eine zentrale Spitze darstellt (3)

5. Das Implantat nach Anspruch 1, **dadurch gekennzeichnet, dass** die Achse des Implantatkörpers eine krumme Linie ist.

6. Das Implantat nach Anspruch 1, **dadurch gekennzeichnet, dass** am Fragment des jeweiligen bionischen Arms (2) eine Erhebung (4) vorkommt.

7. Das Implantat nach Anspruch 1, **dadurch gekennzeichnet, dass** der abgerundete Implantatkörper eine Konkavität (1) aufweist, welche die Funktion einer Kuppel erfüllt, die man mit härtbarem Stoff verfüllen kann, wobei die Fläche des Querschnittes von der Kuppelöffnung kleiner ist als die Oberfläche des Querschnittes von seinem mittigen Teil.

## Revendications

1. Implant bionique sur mesure ou standard, notamment destiné à des applications dentaires, comprenant au moins un ancrage monobloc et au moins une tige arrondie, et qui est muni d'une plate-forme prothétique, l'ancrage comportant au moins deux bras (2), dont les axes sont courbés et s'étendent à partir d'un point situé sur l'axe de la tige, **caractérisé en ce qu'**au moins deux bras (2) sont des bras bioniques (2) destinés à pénétrer dans l'os, qui se rétrécissent de manière circonférentielle, c'est-à-dire en direction de la tige, formant ainsi au moins une lame (6) sur chaque bras, les bras comportant des lames sur leurs bords, et les lames pouvant s'enfoncer (mordre) dans les parois de l'alvéole dentaire, assurant ainsi une stabilité supplémentaire.

2. Implant selon la revendication 1, **caractérisé en ce qu'**au moins deux bras bioniques (2) présentent en outre des extrémités effilées.

3. Implant selon la revendication 1, **caractérisé en ce qu'**au moins deux bras bioniques (2) se rejoignent (s'entrecroisent) pour former un corps intégré.

4. Implant selon la revendication 1, **caractérisé par** un bras supplémentaire se terminant par une pointe acérée (5), qui constitue la pointe centrale (3)

5. Implant selon la revendication 1, **caractérisé en ce que** l'axe du corps de l'implant est une ligne courbe

6. Implant selon la revendication 1, **caractérisé en ce qu'**une protubérance est présente sur une partie de chaque bras bionique (2) (4)

7. Implant selon la revendication 1, **caractérisé en ce que** la tige arrondie comporte une concavité (1) faisant office de dôme, qui peut être remplie d'un matériau durcissable, la surface de section transversale de l'ouverture du dôme étant inférieure à la surface de section transversale de sa partie centrale.
